# EUROPEAN PATENT SPECIFICATION

(11) **EP 3 681 638 B1**
(45) Date of publication and mention of the grant of the patent: **20.04.2022**
(21) Application number: 18856953.7
(22) Date of filing: 13.09.2018
(51) Int. Cl.: B01L 3/00, G01N 33/48, G01N 33/50

(54) **IMPROVED LIQUID TEST SAMPLE DISPENSING DEVICE FOR THE COLLECTION OF LIQUID WASTE AND METHODS OF USE RELATED THERETO**
VERBESSERTE VORRICHTUNG ZUR AUSGABE EINER FLÜSSIGEN PROBE ZUM SAMMELN VON FLÜSSIGEM ABFALL UND VERFAHREN ZUR VERWENDUNG IM ZUSAMMENHANG DAMIT
DISPOSITIF AMÉLIORÉ DE DISTRIBUTION D'ÉCHANTILLONS TESTS LIQUIDES POUR LA COLLECTE DE DÉCHETS LIQUIDES ET PROCÉDÉS D'UTILISATION ASSOCIÉS

(30) Priority: 14.09.2017 US 201762558549 P
(43) Date of publication of application: 22.07.2020
(73) Proprietor: Siemens Healthcare Diagnostics Inc., Tarrytown, NY 10591 (US)
(72) Inventor: ULLAH, Aman, Norton Massachusetts 02766 (US)
(74) Representative: Schweitzer, Klaus
(86) International application number: PCT/US2018/050839
(87) International publication number: WO 2019/055627

(56) References cited:
- EP-B1- 0 483 117
- US-A- 5 102 705
- US-A- 5 460 782
- US-A1- 2012 322 099
- US-A1- 2014 186 235
- US-A1- 2016 199 834
- US-A1- 2016 271 602
- US-B1- 6 261 519

## Description

### TECHNICAL FIELD

The presently disclosed and claimed inventive concept(s) relate to a device(s), and method(s) for collecting liquid waste produced as a by-product in analyte(s) detection assays. More specifically, the presently disclosed and claimed inventive concept(s) relate to an improved device that comprises integrated waste collection micro-cavities for the collection of liquid waste produced as a by-product in analyte(s) detection/diagnostic assays, as well as kits and methods of use related thereto.

### BACKGROUND

Numerous devices, kits, and methods exist for injecting liquid test samples within a reaction vessel for conducting diagnostic assays that detect analytes that may be present in the liquid test samples. Such devices have been proven to be effective in diagnostic assays that detect the presence and quantity of certain analytes indicative of a patient's health, including, but not limited to, glycated hemoglobin (HbA1c), microalbumin and creatinine, and lipid-based analytes, such as cholesterol, triglycerides, and/or high-density lipoproteins. However, at the conclusion of the various diagnostic assays, liquid waste is produced that comprises a mixture of used and left-over reaction reagents (both solid and liquid) and a patient's liquid test sample. This liquid waste must be collected so that it does not leak or spill out of the reaction vessel, as leaking and/or spillage could potentially create a biohazard containment issue. Currently, liquid waste collection is primarily handled via injection devices that comprise separate components which absorb the liquid waste at the conclusion of the diagnostic assay. Such components include, but are not limited to, absorbent tubes, absorbent pads, and/or elastomer seals. Such separate components carry not only a per unit cost, but an additional processing cost associated with the integration of these components into the systems via automated manufacturing equipment. In addition, there is an increased cost associated with the labor and overhead required to run and maintain the manufacturing equipment. Moreover, unnecessary variability can be introduced between liquid waste collection products during the manufacturing process which can result in ineffective or incomplete liquid waste collection.

Accordingly, there is a need for an improved device that dispenses a liquid test sample into a reaction vessel but which comprises a fully-integrated, unitary structure liquid waste collection system. Such improved devices, kits, and methods thereby allow, by way of example, and not by way of limitation, for: (1) increased efficiencies and effectiveness in liquid waste collection; and (2) a decrease in cost(s) and manufacturing overhead associated with the production of a fully-integrated, unitary structure liquid waste collection system. It is to such device(s), kit(s), and method(s) that the presently disclosed and claimed inventive concept(s) is directed.

### DESCRIPTION OF THE SEVERAL VIEWS OF THE DRAWINGS

Figure 1 is a perspective view of one non-limiting embodiment of an improved liquid test sample dispensing device constructed in accordance with the presently disclosed and/or claimed inventive concept(s).
Figure 2 is a perspective view of an alternative non-limiting embodiment of an improved liquid test sample dispensing device constructed in accordance with the presently disclosed and/or claimed inventive concept(s).
Figure 3A is a perspective view of an alternative non-limiting embodiment of an improved liquid test sample dispensing device constructed in accordance with the presently disclosed and/or claimed inventive concept(s).
Figure 3B is a perspective view of the improved liquid test sample dispensing device of FIG. 3A in which the plunger has been inserted into the plunger receptacle of the body.
Figure 4 is a perspective view of the liquid test sample dispensing device of FIG. 1 which has been inserted into a reaction vessel.

### DETAILED DESCRIPTION

The invention is defined in the appended claims. Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary-not exhaustive. Also, it is to be understood that the phraseology and terminology employed herein is for the purpose of description and should not be regarded as limiting.

Unless otherwise defined herein, scientific and technical terms used in connection with the presently disclosed and claimed inventive concept(s) shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art.

All patents, published patent applications, and non-patent publications mentioned in the specification are indicative of the level of skill of those skilled in the art to which this presently disclosed and claimed inventive concept(s) pertains.

All of the devices and/or methods disclosed and claimed herein can be made and executed without undue experimentation in light of the present disclosure.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The singular forms "a," "an," and "the" include plural referents unless the context clearly indicates otherwise. Thus, for example, reference to "a compound" may refer to 1 or more, 2 or more, 3 or more, 4 or more or greater numbers of compounds. The term "plurality" refers to "two or more." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. For example but not by way of limitation, when the term "about" is utilized, the designated value may vary by ± 20% or ± 10%, or ± 5%, or ± 1%, or ± 0.1% from the specified value, as such variations are appropriate to perform the disclosed methods and as understood by persons having ordinary skill in the art. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z. The use of ordinal number terminology (i.e., "first", "second", "third", "fourth", etc.) is solely for the purpose of differentiating between two or more items and is not meant to imply any sequence or order or importance to one item over another or any order of addition, for example.

As used in this specification and claim(s), the terms "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, the term "substantially" means that the subsequently described event or circumstance occurs at least 90% of the time, or at least 95% of the time, or at least 98% of the time.

As used herein, the phrase "associated with" includes both direct association of two moieties to one another as well as indirect association of two moieties to one another. Non-limiting examples of associations include covalent binding of one moiety to another moiety either by a direct bond or through a spacer group, non-covalent binding of one moiety to another moiety either directly or by means of specific binding pair members bound to the moieties, incorporation of one moiety into another moiety such as by dissolving one moiety in another moiety or by synthesis, and coating one moiety on another moiety.

The term "liquid test sample" as used herein will be understood to include any type of biological fluid sample that may be utilized in accordance with the presently disclosed and claimed inventive concept(s). Examples of biological samples that may be utilized include, but are not limited to, whole blood or any portion thereof (i.e., plasma or serum), saliva, sputum, cerebrospinal fluid (CSF), intestinal fluid, intraperotineal fluid, cystic fluid, sweat, interstitial fluid, tears, mucus, urine, bladder wash, semen, combinations, and the like. As used herein, the term "volume" as it relates to the liquid test sample utilized in accordance with the presently disclosed and claimed inventive concept(s) means from about 0.1 microliter to about 100 microliters, or from about 1 microliter to about 75 microliters, or from about 2 microliters to about 60 microliters, or less than or equal to about 50 microliters, or less than or equal to about 40 microliters. For example, the liquid test sample is either about 1 microliter of whole blood and/or about 40 microliters of urine.

The term "liquid waste" as used herein means a combination of a patient's liquid test sample and liquid (and/or solid) reagents utilized to conduct at least one diagnostic assay. In one non-limiting embodiment of the presently disclosed and/or claimed inventive concept(s), the liquid waste is contained within the reaction vessel immediately after the conductance of the at least one diagnostic assay and prior to the collection of the liquid waste within the plurality of micro-cavities formed on at least one side of the body of the liquid sample dispensing device. As used herein, the term "volume," as it relates to the liquid waste produced in accordance with the presently disclosed and/or claimed inventive concept(s), means from about 1 microliter to about 700 microliters, or from about 10 microliters to about 650 microliters, or from about 25 microliters to about 600 microliters, or from about 50 microliters to about 550 microliters, or from about 100 microliters to about 500 microliters, or from about 150 microliters to about 450 microliters, or from about 200 to about 400 microliters, or from about 250 microliters to about 350 microliters, or greater than or equal to about 300 microliters. In one non-limiting embodiment, the volume of liquid waste is about 600 microliters.

The terms "capillary action" and/or "capillary force" as used herein will be understood to include the interaction between contacting surfaces of a liquid and a solid that distorts the liquid surface from a planar shape and causes the liquid rise, fall, or remain contained in a narrow tube, channel, and/or cavity. By way of example only, and not by way of limitation, capillary action includes: (1) the wicking of the liquid test sample into the capillary of the liquid sample dispensing device, such that liquid test sample remains in the capillary until agitated; and (2) the wicking in and containment of the liquid waste within the plurality of micro-cavities.

The term "hydrophilic coating(s)" as used herein will be understood to mean any substance(s) that reduce surface friction and enhance the lubricity of an object. Hydrophilic coating(s), as used herein, can be applied to either (1) the inside and/or outside surface(s) of the capillary to enhance the collection of a patient's liquid test sample via capillary action into the capillary; and/or (2) the internal surfaces of some or all of the micro-cavities comprising the plurality of micro-cavities to thereby enhance the collection and containment of liquid waste produced as a by-product of conducting at least one diagnostic assay within the plurality of micro-cavities. By way of example only, and not by way of limitation, examples of hydrophilic coating(s) utilized in accordance with the presently disclosed and/or claimed inventive concept(s) include, but are not limited to, polyvinylpyrolidone (PVP), polyurethanes, polyacrylic acid (PAA), polyethylene oxide (PEO), polysaccharide materials, organic and/or inorganic polymeric materials, and combinations thereof.

The term "patient" includes human and veterinary subjects. In certain examples, a patient is a mammal. In certain other examples, the patient is a human. "Mammal" for purposes of treatment refers to any animal classified as a mammal, including human, domestic and farm animals, nonhuman primates, and zoo, sports, or pet animals, such as dogs, horses, cats, cows, etc.

The term "reaction vessel" includes any device(s) capable of performing at least one diagnostic assay as described herein. The reaction vessel may perform the diagnostic assay(s) manually, but, in most instances, the reaction vessel will be inserted into a system that automates the performance of the diagnostic assay(s). For example, the reaction vessel comprises a reaction cassette for use in automated diagnostic assays conducted by the DCA Vantage^{®} Analyzer commercially available from Siemens Healthcare Diagnostics, Inc.

Turning now to particular embodiments, the presently disclosed and claimed inventive concept(s) relate to a device(s) and method(s) for injecting a patient's liquid test sample into a reaction vessel and for the subsequent collection of liquid waste produced as a by-product from conducting a diagnostic assay. More specifically, the presently disclosed and claimed inventive concept(s) relate to an improved liquid test sample injection device that comprises a plurality of micro-cavities for the collection of the liquid waste produced from a diagnostic assay, and methods of use related thereto.

It is contemplated that virtually any reagent used in the fields of biological, chemical, or biochemical analyses and assays could be used in the devices and methods of the presently claimed and disclosed inventive concept(s). It is contemplated that these reagents may undergo physical and/or chemical changes when bound to an analyte of interest whereby the intensity, nature, frequency, or type of signal generated by the reagent-analyte complex is directly proportional or inversely proportional to the concentration of the analyte existing within the fluid sample. These reagents may contain indicator dyes, metal, enzymes, polymers, antibodies, and electrochemically reactive ingredients and/or chemicals that, when reacting with an analyte(s) of interest, may exhibit change in color.

Any method of detecting and measuring the analyte in a fluid sample can be used in the devices and methods of the presently claimed and inventive concepts. A variety of assays for detecting analytes are well known in the art and include, but are not limited to, chemical assays, enzyme inhibition assays, antibody stains, latex agglutination, latex agglutination inhibition and immunoassays, such as, radioimmunoassays. The term "antibody" herein is used in the broadest sense and refers to, for example, intact monoclonal antibodies, polyclonal antibodies, multi-specific antibodies (e.g., bispecific antibodies), and to antibody fragments that exhibit the desired biological activity (e.g., antigen/analyte-binding). The antibody can be of any type or class (e.g., IgG, IgE, IgM, IgD, and IgA) or sub-class (e.g., IgG1, IgG2, IgG3, IgG4, IgA1, and IgA2).

While immunoassays (including, but not limited to, sequential analytical chemical and immunoassays) are primarily discussed herein for the detection of at least one analyte of interest present in a liquid test sample, a person having ordinary skill in the art should readily understand that the presently disclosed and claimed inventive concept(s) are not strictly limited to immunoassays and may include, by way of example and not by limitation, chemical and chemical-based assays, nucleic acid assays, lipid-based assays, and serology-based assays. Immunoassays, including radioimmunoassays and enzyme-linked immunoassays, are useful methods for use with the presently claimed and disclosed inventive concepts. A variety of immunoassay formats, including, for example, competitive and non-competitive immunoassay formats, antigen/analyte capture assays and two-antibody sandwich assays can be used in the methods of the invention. Enzyme-linked immunosorbent assays (ELISAs) can be used in the presently claimed and disclosed inventive concepts, as well. In the case of an enzyme immunoassay, an enzyme is typically conjugated to a second antibody, generally by means of glutaraldehyde, periodate, hetero-bifunctional crosslinking agents, or biotin-streptavidin complexes. As will be readily recognized, however, a wide variety of different conjugation techniques exist which are readily available for use with the presently disclosed and claimed inventive concept(s) to one skilled in the art.

Assays, including, but not limited to, immunoassays, nucleic acid capture assays, lipid-based assays, and serology-based assays, can be developed for a multiplexed panel of proteins, peptides, and nucleic acids which may be contained within a liquid test sample, with such proteins and peptides including, for example but not by way of limitation, albumin, microalbumin, cholesterol, triglycerides, high-density lipoproteins, low-density lipoproteins, hemoglobin, myoglobin, α-1-microglobin, immunoglobins, enzymes, proteins, glycoproteins, protease inhibitors, drugs, cytokines, creatinine, and glucose. The device(s), kit(s), and method(s) disclosed and/or claimed herein may be used for the analysis of any liquid test sample, including, without limitation, whole blood, plasma, serum, or urine.

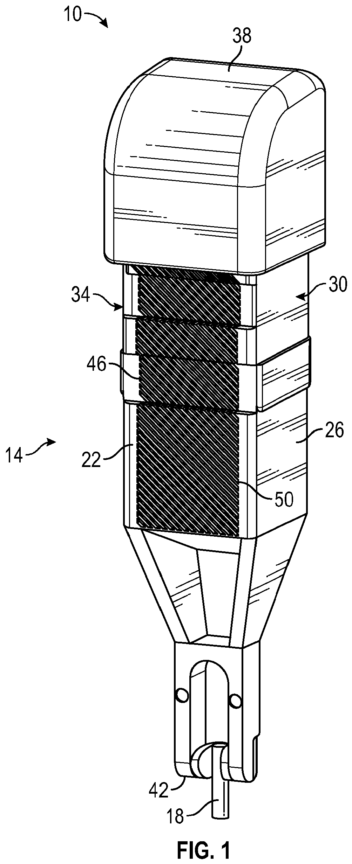

Referring now to the Figures, and more particularly to FIG. 1, shown therein is a non-limiting embodiment of a liquid test sample dispensing device 10 that both dispenses a patient's liquid test sample into a reaction vessel and collects liquid waste following the conductance of at least one diagnostic assay. The liquid test sample dispensing device 10 comprises a body 14 and a capillary 18.

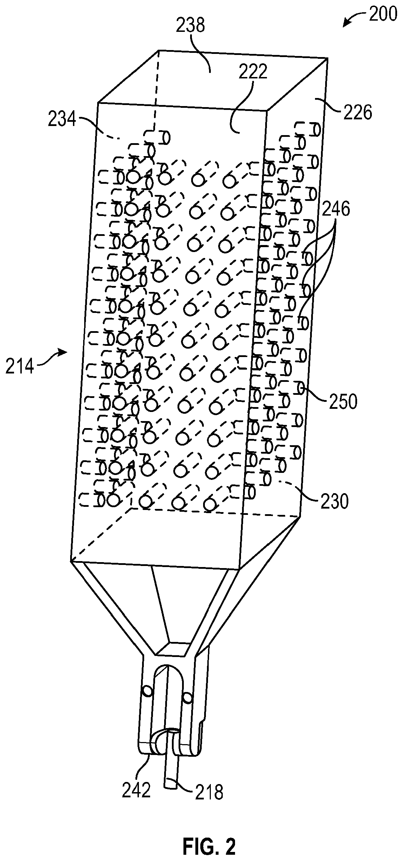

In one non-limiting embodiment, and as shown in FIG. 1, the body 14 comprises a first side 22, a second side 26, a third side 30, a fourth side 34, a first end 38, a second end 42, and a plurality of micro-cavities 46. While depicted in FIG. 1 as comprising four sides, it should be readily understood that the body 14 (or any other embodiment of the body contained herein) can comprise any number of sides that accomplishes the presently disclosed and/or claimed inventive concept(s), including, without limitation, the body 14 may comprise 1, 2, 3, 4, 5, 6, 7, 8, 9, 10, 11, 12, 13, 14, 15, 16, 17, 18, 19, 20, 30, 40, 50, 60, 70, 80, 90, or greater than or equal to 100 sides. In addition, while shown in FIG. 1 as being substantially rectangular in shape, the body 14 can be configured to be any shape that accomplishes the presently disclosed and/or claimed inventive concept(s), including, without limitation, circular, ovular, triangular prism, cube, rectangular prism, trapezoidal prism, pentagonal prism, hexagonal prism, heptagonal prism, octagonal prism, nonagonal prism, decagonal prism, or any polygonal prism (in which case, the number of sides will comport with the particular shape of the body 14-i.e., an octagonal prism-shaped body 14 comprises eight sides/faces). As shown in greater detail in FIG. 2, the body 14 of the liquid test sample dispensing device 10 is configured so as to be received in a reaction vessel wherein a patient's liquid test sample is dispensed from the capillary 18 into the reaction vessel for the conductance of at least one diagnostic assay, such as, by way of example only, a glycated hemoglobin diagnostic assay or a creatinine diagnostic assay.

In certain non-limiting embodiments, the body 14 is fabricated as a molded, unitary component formed of a rigid plastic material (so as to avoid deformation of the body 14 when both collecting a patient's liquid test sample and/or inserting the body into a reaction vessel), including, for example, synthetic and/or naturally-occurring or derived polymers (both organic and/or inorganic), such as, by way of example only, thermoplastic polymer(s), thermoset polymer(s), elastomer(s), and/or synthetic fiber(s) such as low-density polyethylene, high-density polyethylene, polystyrene, polyvinylchloride, styrene butadiene, acrylic(s), polyacrylics, polyvinyl acetate, and combinations thereof.. However, a person having ordinary skill in the art should readily appreciate that the body 14 may be constructed of any material capable of accomplishing the presently disclosed and/or claimed inventive concept(s). In one non-limiting embodiment (as shown in FIG. 1), the body 14 of the liquid test sample dispensing device 10 is constructed such that the area defined by the first side 22, second side 26, third side 30, and fourth side 34 of the body 14 is solid, with no hollow spaces existing in such area. However, the area defined by the first side 22, second side 26, third side 30, and fourth side 34 of the body 14 need not be entirely solid and may contain at least a portion of the area that includes a hollow space-such configuration would allow for savings in manufacturing costs due to less material usage.

As shown in FIG. 1, the plurality of micro-cavities 46 are disposed on and formed in the first side 22 of the body 14; however, it should be readily understood to a person having ordinary skill in the art that the plurality of micro-cavities 46 can be located on any side or sides comprising the body 14. In one non-limiting embodiment, the plurality of micro-cavities are disposed on and formed in each of the first side 22, second side 26, third side 30, and fourth side 34 of the body 14. Similarly, when the body 14 comprises a single side or at least one side (for instance, when the body 14 is circular in shape), the plurality of micro-cavities 46 may be disposed on and formed in either a portion of the at least one side of the body 14, or alternatively the plurality of micro-cavities 46 may be disposed on and formed in the entirety of the at least one side. In addition, the plurality of micro-cavities 46 is substantially located between the first end 38 and the second end 42 of the body 14.

The plurality of micro-cavities 46 may be formed in the side(s) of the body 14 via any manufacturing techniques commonly known in the art, including, without limitation, injection molding, thermoforming, compression molding, 3-dimensional printing, turning, drilling, milling, etching, and combinations thereof. In one non-limiting embodiment, each micro-cavity of the plurality of micro-cavities 46 comprise an opening 50, the opening 50 being located on an outer surface (not numbered) of the at least one side of the body 14 (for example, the first side 22). The plurality of micro-cavities 46 can be of any configuration which allows the liquid waste produced from a diagnostic assay (such as, by way of example only, a glycated hemoglobin assay or a microalbumin/creatinine assay) to enter into the plurality of micro-cavities 46 via capillary action through the opening(s) 50 and remain within the plurality of micro-cavities 46. For instance, as shown in FIG. 1, the plurality of micro-cavities 46 may be configured such that the opening(s) 50 extend along the entirety of the first side 22 (or any and/or all sides) of the body 14 forming substantially parallel, groove-like structures located within the first side 22 of the body 14. Alternatively, as shown in greater detail in FIG. 2, the plurality of micro-cavities 46 may be configured so that the opening(s) 50 are located on an outer surface(s) (not numbered) of the first side 22, second side 26, and third side 30 of the body 14 such that each micro-cavity of the plurality of micro-cavities 46 extends inward from the opening(s) and is oriented substantially perpendicular to the respective side of the body 14 for which the opening(s) 50 are located. A person having ordinary skill in the art should readily understand that the plurality of micro-cavities 46 can be formed within any or all of the side(s) comprising the body 14.

The liquid waste, upon contacting the opening(s) 50, flows into the plurality of micro-cavities 46 via capillary action. Accordingly, the liquid waste is retained within the plurality of micro-cavities 46 due to the adhesion between the liquid waste and the solid, inner surface (not numbered) of the plurality of micro-cavities 46 and/or the surface tension of the liquid waste created by the cohesion of the molecules comprising the liquid waste. As a result of the capillary action and retention of the liquid waste within the plurality of micro-cavities 46, the liquid waste is collected within the plurality of micro-cavities 46 of the body 14 of the liquid sample dispensing device 10. This retention allows for the safe, effective, and efficient disposal of the liquid waste while mitigating and/or eliminating the spillage of the liquid waste that could potentially create a biohazard situation.

Each micro-cavity of the plurality of micro-cavities 46 comprises a substantially three-dimensional, geometric shape. Each micro-cavity of the plurality of micro-cavities 46 may be the same shape, different shapes, or a combination of the same and different shapes. Three-dimensional shapes of each micro-cavity utilized in accordance with the presently disclosed and/or claimed inventive concept(s) include, but are not limited, a sphere, cylinder, cone, triangular prism, tetrahedron, square pyramid, cube, rectangular prism (including, without limitation, a herringbone orientation), pentagonal prism, hexagonal prism, heptagonal prism, octagonal prism, hendecagonal prism, dodecahedral prism, and any combinations thereof. For purposes of clarity, each micro-cavity of the plurality of micro-cavities 46 may comprise any polygonal prism. Each micro-cavity of the plurality of micro-cavities 46 can be of any dimension(s) that allows for the liquid waste to be drawn into such plurality of micro-cavities 46 via capillary action and retained therein once drawn into the plurality of micro-cavities 46. By way of example only, the plurality of micro-cavities may comprise a radius and/or apothem (depending on the shape(s) comprising the plurality of micro-cavities 46) of about 0.1 millimeter to about 2 millimeters, or from about 0.2 millimeter to about 1.9 millimeters, or from about 0.3 millimeter to about 1.8 millimeters, or from about 0.4 millimeters to about 1.7 millimeters, or from about 0.5 millimeter to about 1.6 millimeters, or from about 0.6 millimeter to about 1.5 millimeters, or from about 0.7 millimeters to about 1.4 millimeter, or from about 0.8 millimeter to about 1.4 millimeter, or from about 0.9 millimeter to about 1.3 millimeters, or from about 1 millimeter to about 1.2 millimeters, or equal to about 1.1 millimeters. In one non-limiting embodiment, the radius and/or apothem of each micro-cavity of the plurality of micro-cavities is from about 1.2 millimeters to about 1.3 millimeters. In addition, some or all of the micro-cavities of the plurality of micro-cavities 46 may further comprise at least one hydrophilic coating(s).

The capillary 18 is adapted to collect a patient's liquid test sample and to subsequently inject the liquid test sample into a reaction vessel. In one non-limiting embodiment, the capillary 18 collects the patient's liquid fluid sample via capillary action when the capillary 18 is in contact with the patient's liquid test sample. However, a person having ordinary skill in the art should readily appreciate that the liquid test sample can be collected by the capillary 18 via any method commonly known in the art, including, without limitation, via creation of a negative pressure differential that draws the patient's liquid test sample into the capillary 18. The capillary 18 can be constructed of any material(s) commonly known in the art, including, without limitation, glass and/or chemically-inert plastic(s). The size and volume-capacity of the capillary 18 will vary depending on the type and quantity of the patient's fluid sample being collected. The capillary 18 may be adapted and sized to hold volumes of from about 0.1 microliter to about 100 microliters, or from about 0.5 microliters to about 95 microliters, or from about 1 microliter to about 90 microliters, or from about 2 microliters to about 85 microliters, or from about 5 microliters to about 80 microliters, or from about 10 microliters to about 75 microliters, or from about 15 microliters to about 70 microliters, or from about 20 microliters to about 65 microliters, or from about 25 microliters to about 60 microliters, or from about 30 microliters to about 55 microliters, or from about 35 to about 50 microliters, or less than or equal to about 40 microliters. By way of example only, and not by way of limitation, the volume capacity of the capillary 18 is about 1 microliter when the patient's liquid test sample is whole blood. In one non-limiting embodiment, the capillary 18 extends through the second end 42 of the body 14 wherein at least a portion of the capillary 18 remains external to the body 14 for collection of the patient's liquid test sample. In addition, the capillary 18 may further comprise at least one hydrophilic coating(s).

Referring now to FIG. 2, shown therein is a perspective view of an alternative non-limiting embodiment of an improved liquid test sample dispensing device 200 constructed in accordance with the presently disclosed and/or claimed inventive concept(s). The description with respect to the improved liquid test sample dispensing device 10 of FIG. 1 is equally applicable to the improved liquid test sample dispensing device 200 depicted in FIG. 2, the notable difference being the orientation of the plurality of micro-cavities 246. Accordingly, for purposes of brevity, only the differences, if any, between the embodiments will be discussed hereinbelow.

As shown in FIG. 2, the improved liquid test sample dispensing device 200 comprises a body 214 and a capillary 218.

In one non-limiting embodiment, and as shown in FIG. 2, the body 214 comprises a first side 222, a second side 226, a third side 230, a fourth side 234, a first end 238, a second end 242, and a plurality of micro-cavities 246. As shown in FIG.2, the plurality of micro-cavities 246 are disposed on and formed in the first side 222, the second side 226, and the fourth side 234 of the body 214; however, it should be readily understood to a person having ordinary skill in the art that the plurality of micro-cavities 246 (and the opening(s) 250) can be located on any side or side comprising the body 214. Each micro-cavity of the plurality of micro-cavities 246 comprise an opening(s) 250, the opening(s) 250 of each micro-cavity being located on an outer surface (not numbered) of the side or sides comprising the body 214. The plurality of micro-cavities 46 are configured so that the opening(s) 250 are located on the outer surface(s) (not numbered) of the first side 222, the second side 226, and the fourth side 234 of the body 214 such that each micro-cavity of the plurality of micro-cavities 246 extends inward into the body 214 away from the opening(s) 250 so that each micro-cavity is substantially perpendicular to the respective side of the body 214 from which the opening(s) 250 are located; however, it should be readily understood to a person having ordinary skill in the art that each micro-cavity of the plurality of micro-cavities can extend inward at any angle (including, the same, different, or combination of same and different angles) that allows for capillary action to occur. Each micro-cavity of the plurality of micro-cavities 246 extends inward into the body 214 to a length (as measured from the respective opening 250 on a side of the body 214) of from about 0.1 millimeter to about 5 millimeters, or from about 0.2 millimeter to about 4.9 millimeters, or from about 0.3 millimeter to about 4.8 millimeters, or from about 0.4 millimeter to about 4.7 millimeters, or from about 0.5 millimeter to about 4.6 millimeters, or from about 0.6 millimeter to about 4.5 millimeters, or from about 0.7 millimeter to about 4.4 millimeters, or from about 0.8 millimeter to about 4.3 millimeters, or from about 0.9 millimeter to about 4.2 millimeters, or from about 1 millimeter to about 4.1 millimeters, or from about 1.1 millimeters to about 4.0 millimeters, or from about 1.2 millimeters to about 3.9 millimeters, or from about 1.3 millimeters to about 3.8 millimeters, or from about 1.4 millimeters to about 3.7 millimeters, or from about 1.5 millimeters to about 3.6 millimeters, or from about 1.6 millimeters to about 3.5 millimeters, or from 1.7 millimeters to about 3.4 millimeters, or from about 1.8 millimeters to about 3.3 millimeters, or from about 1.9 millimeters to about 3.2 millimeters, or from about 2 millimeters to about 3.1 millimeters, or from about 2.1 millimeters to about 3 millimeters, or from about 2.2 millimeters to about 2.9 millimeters, or from about 2.3 millimeters to about 2.8 millimeters, or from about 2.4 millimeters to about 2.7 millimeters, or from about 2.5 millimeters to about 2.6 millimeters. In one non-limiting embodiment, the each micro-cavity extends a length of from about 4.5 millimeters to about 5 millimeters.

While shown in FIG. 2 as being evenly spaced on a side (i.e., the first side 222, the second side 226, and the fourth side 234) of the body 214, it should be readily understood that each micro-cavity of the plurality of micro-cavities 246 (and their respective openings 250) need not be evenly spaced and can be oriented in any manner on (or within) a side of the body 214 that accomplishes the presently disclosed and/or claimed inventive concept(s). Similarly, while shown in FIG. 2 as comprising the same length, it should be readily understood to a person having ordinary skill in the art that the micro-cavities comprising the plurality of micro-cavities 246 can be the same length, different lengths, or a combination of the same and different lengths. Likewise, while depicted in FIG. 2 as comprising forty-four (44) micro cavities per each side of the first side 222, the second side 226, and the fourth side 234 of the body 214, it should be readily understood to a person having ordinary skill in the art that the body 214 of the improved liquid test sample dispensing device 200 may comprise any number of micro-cavities that accomplishes the presently disclosed and/or claimed inventive concept(s), including, by way of example only, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, or greater than or equal to 1,000 micro-cavities. In addition, as previously stated herein, the micro-cavities can be located in (and/or on) any or all of the side(s) comprising the body 214 of the improved liquid test sample dispensing device 200. In addition, some or all of the micro-cavities of the plurality of micro-cavities 46 may further comprise at least one hydrophilic coating(s).

The liquid waste, upon contacting the opening(s) 250, flows into the plurality of micro-cavities 246 via capillary action. Accordingly, the liquid waste is retained within the plurality of micro-cavities 246 due to the adhesion between the liquid waste and the solid, inner surface (not numbered) of the plurality of micro-cavities 246 and/or the surface tension of the liquid waste created by the cohesion of the molecules comprising the liquid waste. As a result of the capillary action and retention of the liquid waste within the plurality of micro-cavities 246, the liquid waste is collected within the plurality of micro-cavities 246 of the body 214 of the liquid sample dispensing device 200. This retention allows for the safe, effective, and efficient disposal of the liquid waste while mitigating and/or eliminating the spillage of the liquid waste that could potentially create a biohazard situation.

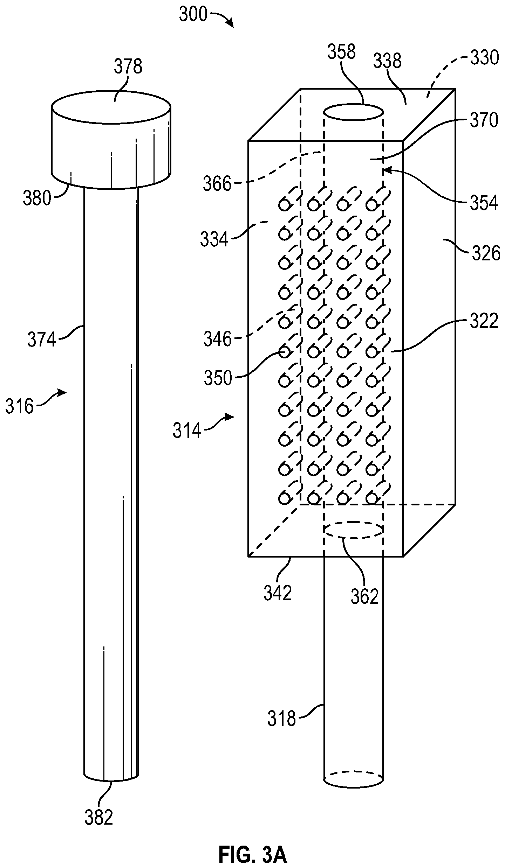

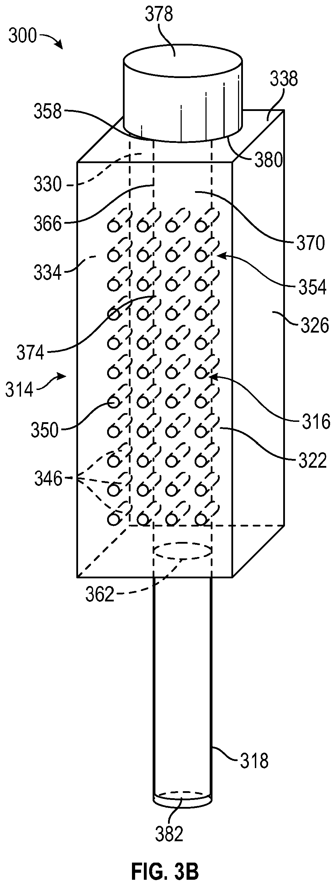

Referring now to FIGS. 3A and 3B, shown therein is perspective view of an alternative non-limiting embodiment of an improved liquid test sample dispensing device 300 constructed in accordance with the presently disclosed and/or claimed inventive concept(s). The descriptions with respect to FIGS. 1 and 2 are equally applicable to the device 300 depicted in FIGS. 3A and 3B, the notable difference being the liquid test sample dispensing device 300 additionally comprises a plunger 316 and a plunger receptacle 354. Accordingly, for purposes of brevity, only the differences, if any, between the embodiments will be discussed hereinbelow.

As shown in FIG. 3A, the improved liquid test sample dispensing device 300 comprises a body 314, a plunger 316, and a capillary 318.

In one non-limiting embodiment, and as shown in FIG. 3A, the body 314 comprises a first side 322, a second side 326, a third side 330, a fourth side 334, a first end 338, a second end 342, a plurality of micro-cavities 346 (wherein each of the micro-cavities of the plurality of micro-cavities 346 comprises an opening 350), and a plunger receptacle 354. The plunger receptacle 354 comprises a receptacle wall (not numbered) having a first end 358, a second end 362, at least one side 366, and a bore 370 extending longitudinally from the first end 358 to the second end 362 of the receptacle wall of the plunger receptacle 354, such that the bore 370 is substantially parallel to and contained between the first side 322, the second side 326, the third side 330, and the fourth side 334 of the body 314. The bore 370 can be configured to be any shape, including, without limitation, cylindrical, pyramidal, cube, rectangular prism, trapezoidal prism, pentagonal prism, hexagonal prism, heptagonal prism, octagonal prism, nonagonal prism, decagonal prism, or any polygonal prism. In one configuration, the bore 370 is cylindrical. The plunger receptacle 354 is configured to receive the plunger 316 (shown in greater detail in FIG. 3B) through an opening (not numbered) located at the first end 358 of the plunger receptacle 354.

While shown in FIG. 3A (and FIG. 3B) as comprising forty four (44) micro-cavities extending inwardly solely from the first side 322 of the body 314, it should be readily understood to a person having ordinary skill in the art (and as previously discussed herein with respect to FIG. 2) that the plurality of micro-cavities 346 can comprise any number of micro-cavities capable of accomplishing the presently disclosed and/or claimed inventive concept(s). In addition, while shown in FIG. 3A (and FIG. 3B) as only be located on and within the first side 322 of the body 314, a person having ordinary skill in the art should readily appreciate that the plurality of micro-cavities 346 can be located on and in any or all of the side(s) comprising the body 314. In addition, some or all of the micro-cavities of the plurality of micro-cavities 46 may further comprise at least one hydrophilic coating(s).

The plunger 316 comprises a plunger body 374 having a proximal handle end 378 and a distal end 382. The proximal handle end 378 may comprise at least one flange 380 for selectively securing the plunger 316 within the plunger receptacle 354 when the plunger 316 is inserted into the plunger receptacle 354. The plunger 316 is preferably constructed of a semi-rigid, inert material(s) that allows(s) for the plunger 316 to be easily inserted into the plunger receptacle 354 with minimal, if any, deformation and/or bending of the plunger 316 upon insertion of the plunger 316 into the plunger receptacle 354. Suitable materials for constructing the plunger 316, include, without limitation, synthetic and/or naturally-occurring or derived polymers (both organic and/or inorganic), such as, by way of example only, thermoplastic polymer(s), thermoset polymer(s), elastomer(s), and/or synthetic fiber(s) such as low-density polyethylene, high-density polyethylene, polystyrene, polyvinylchloride, styrene butadiene, acrylic(s), polyacrylics, polyvinyl acetate, and combinations thereof.

The capillary 318 is adapted to collect a patient's liquid test sample and to subsequently inject the liquid test sample into a reaction vessel. In one non-limiting embodiment, the capillary 318 collects the patient's liquid test sample via capillary action when the capillary 318 is in contact with the patient's liquid test sample. However, a person having ordinary skill in the art should readily appreciate that the liquid test sample can be collected by the creation of a negative pressure differential that draws the patient's liquid test sample into the capillary 318. The capillary 318 can be constructed of any material(s) commonly known in the art, including, without limitation, glass and/or chemically-inert plastic(s). The size and volume capacity of the capillary 318 will vary depending on the type and quantity of the patient's liquid test sample being collected. The capillary 318 may be adapted and sized to hold volumes of from about 0.1 microliter to about 100 microliters, or from about 0.5 microliter to about 95 microliters, or from about 1 microliter to about 90 microliters, or from about 2 microliters to about 85 microliters, or from about 5 microliters to about 80 microliters, or from about 10 microliters to about 75 microliters, or from about 15 microliters to about 70 microliters, or from about 20 microliters to about 65 microliters, or from about 25 microliters to about 60 microliters, or from about 30 microliters to about 55 microliters, or from about 35 microliters to about 50 microliters, or less than or equal to about 40 microliters. By way of example only, and not by way of limitation, the volume capacity of the capillary 318 is about 1 microliter when the patient's liquid test sample is whole blood and about 40 microliters when the patient's liquid test sample is urine. In one configuration, (and as shown in FIGS. 3A and 3B), the capillary 318 and the plunger receptacle 354 comprise a unitary structure-the capillary 318 extending externally from the body 314 from the second end 342 of the body 314 in order to collect the patient's liquid test sample. Alternatively, the capillary 318 may be a separate structure from the plunger receptacle 354. In this configuration, the capillary 318 extends through the second end 342 of the body 314 and the second end 362 of the plunger receptacle 354 such that a portion of the capillary 318 is positioned and secured within the plunger receptacle 354, while at least a portion of the capillary 318 remains external to the body 314 for the collection of the patient's liquid test sample. In addition, the capillary 318 may further comprise at least one hydrophilic coating(s).

Referring now to FIG. 3B, shown therein is a perspective view of the improved liquid test sample dispensing device 300 in which the plunger 316 has been inserted into and secured within the plunger receptacle 354 of the body 314. While FIG. 3B depicts the plunger 316 having already been inserted into the plunger receptacle 354, it should be readily understood to a person having ordinary skill in the art that the depression of the plunger 316 occurs either manually or via an automated procedure after the improved liquid test sample dispensing device 300 has been secured within a reaction vessel to thereby accomplish the presently disclosed and/or claimed inventive concept(s). Upon insertion of the plunger 316 into the plunger receptacle 354 of the body 314 (which has already occurred in FIG. 3B), the downward insertion of the plunger 316 forms a substantially air-tight seal between the plunger body 374 and the at least one side 366 of the receptacle wall of the plunger receptacle 354. The downward motion of the plunger 316 creates a force that produces a pressure sufficient enough to actively and fully inject the patient's liquid test sample from the capillary 318 into a reaction chamber of the reaction vessel for conducting at least one diagnostic assay.

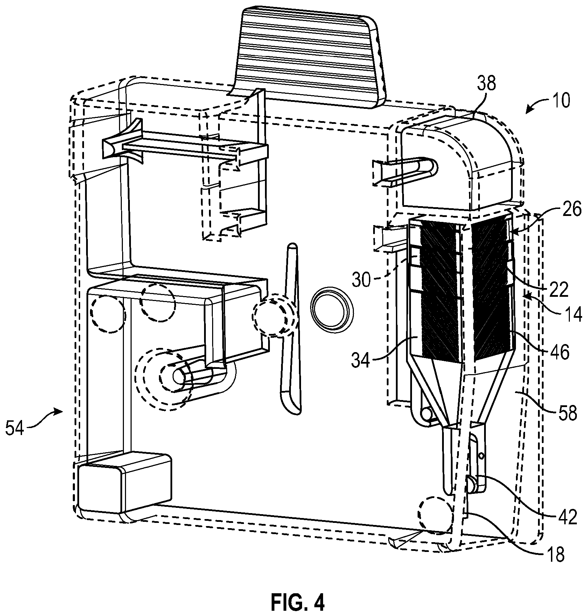

Referring now to FIG. 4, shown therein is a perspective view of the liquid test sample dispensing device 10 of FIG. 1 which has been inserted into a reaction chamber 58 of a reaction vessel 54 for the conductance of at least one diagnostic assay and the subsequent collection of liquid waste produced as a by-product of conducting the at least one diagnostic assay.

Following the collection of a patient's liquid test sample into the capillary 18 of the liquid test sample dispensing device 10, the liquid test sample dispensing device 10 is inserted into and secured within a reaction chamber 58 of a reaction vessel 54. In one example, the reaction vessel is then secured within a diagnostic assay system/instrument (such as, by way of example only, the DCA Vantage^{®} Analyzer commercially available from Siemens Healthcare Diagnostics, Inc.). Once secured within the diagnostic assay system/instrument, the patient's liquid test sample is then dispensed from the capillary 18 into the reaction chamber 58 of the reaction vessel 54 via, for example, the automated rotation and agitation of the reaction vessel 54 within the diagnostic assay system/instrument. Following the dispensing of the patient's liquid test sample into the reaction chamber 58 of the reaction vessel 54, at least one diagnostic assay (such as, by way of example only, a glycated hemoglobin diagnostic assay and/or a microalbumin/creatinine diagnostic assay) is conducted on the patient's liquid test sample. In one example, the at least one diagnostic assay involves pre-determined steps in which the reaction vessel 54 is rotated in both clockwise and counter clockwise directions such that the patient's liquid test sample is sufficiently mixed with both solid and liquid reagents, while various measurements are taken at pre-determined intervals during the conductance of the at least one diagnostic assay. At the conclusion of the at least one diagnostic assay, a volume of liquid waste is contained within the reaction vessel 54, the volume of liquid waste primarily comprising a mixture of the patient's liquid test sample, the solid reagent(s), and/or the liquid reagent(s) utilized in the conductance of the at least one diagnostic assay. Subsequent to the conclusion of the at least one diagnostic assay, the reaction vessel 54 containing the volume of liquid waste is substantially inverted via, for example, rotation of the reaction vessel 54 within the diagnostic assay system/instrument. This inversion allows the volume of liquid waste to come into contact with the opening(s) 50 of the plurality of micro-cavities 46, such that the volume of liquid waste is drawn into and contained/maintained within the plurality of micro-cavities 46 via capillary action. Accordingly, the volume of liquid waste is collected and maintained within the plurality of micro-cavities 46 thereby allowing for the safe removal of the reaction vessel 54 from the diagnostic assay system/instrument and the subsequent discarding of the reaction vessel 54 into an appropriate waste receptacle-while simultaneously avoiding a potential biohazard event resulting from the leakage and/or spillage of the volume of liquid waste from the reaction vessel 54.

Thus, in accordance with the presently disclosed and claimed inventive concept(s), there have been provided devices and methods for collection of liquid waste produced as a by-product of conducting at least one diagnostic assay on a patient's liquid test sample within a reaction vessel. As described herein, the presently disclosed and claimed inventive concept(s) relate to embodiments of an improved liquid test sample injection device for use in the injection of patients' liquid test samples into a reaction vessel for conducting diagnostic assays followed by subsequent collection of the liquid waste produced from such diagnostic assays. Such presently disclosed and/or claimed inventive concept(s) fully satisfy the objectives and advantages set forth hereinabove. Although the presently disclosed and claimed inventive concept(s) has been described in conjunction with the specific drawings, experimentation, results and language set forth hereinabove, it is evident that many alternatives, modifications, and variations will be apparent to those skilled in the art.

## Claims

1. A liquid test sample dispensing device, comprising:
a body (14), the body (14) comprising at least one side, a first end (38), a second (42) end, and a plurality of micro-cavities (46), wherein the plurality of micro-cavities (46) is formed in the at least one side of the body (14), further wherein each micro-cavity (46) of the plurality of micro-cavities (46) comprises an opening (50) located on an outer portion of the at least one side of the body (14), the plurality of micro-cavities (46) being substantially located between the first end (38) and the second end (42) of the body (14); and
a capillary (18) for collecting a patient's liquid test sample and dispensing the patient's liquid test sample into a reaction vessel for the conductance of at least one diagnostic assay, wherein a portion of the capillary (18) is disposed through the second end (42) of the body (14).

2. A liquid test sample dispensing device, comprising:
a plunger (316), the plunger comprising a plunger body (374);
a body (314), the body (314) comprising at least one side and a plunger receptacle (354) configured to receive the plunger body (374), a receptacle wall having at least one side, a first end (354), a second end (362), and a bore (370) longitudinally extending from the first end (354) to the second end (362) of the receptacle wall, the body (314) further comprising a plurality of micro-cavities (346), wherein each micro-cavity (346) of the plurality of micro-cavities (346) comprises an opening (350) located on an outer portion of the at least one side of the body (314), further wherein the plurality of micro-cavities (346) are formed in the at least one side of the body (314) such that the plurality of micro-cavities (346) extend inwardly into the body (314) from the at least one side of the body (314) and the plurality of micro-cavities (346) are substantially located between the at least one side of the body (314) and the at least one side of the receptacle wall; and
a capillary (318) for collecting a patient's liquid test sample and dispensing the patient's liquid test sample into a reaction vessel for the conductance of at least one diagnostic assay, wherein a portion of the capillary (318) is disposed through and external to the second end (362) of body (314).

3. The liquid test sample dispensing device of claim 1 or 2, wherein the patient's liquid test sample is selected from the group consisting of whole blood and urine.

4. The liquid test sample dispensing device of claim 1 or 2, wherein the diagnostic assay is selected from the group consisting of a glycated hemoglobin diagnostic assay and a creatinine diagnostic assay.

5. The liquid test sample dispensing device of claim 1 or 2, wherein the body (14)/ (314) and capillary (18)/(318) are constructed of a material selected from the group consisting of synthetic, naturally-occurring, or derived polymers, organic polymers, inorganic polymers, thermoplastic polymer(s), thermoset polymer(s), elastomer(s), synthetic fiber(s), low-density polyethylene, high-density polyethylene, polystyrene, polyvinylchloride, styrene butadiene, acrylic(s), polyacrylics, polyvinyl acetate, and combinations thereof.

6. The liquid test sample dispensing device of claim 1 or 2, wherein the micro-cavities (46)/(346) are formed in the at least one side of the body (14)/ (314) via manufacturing techniques selected from the group consisting of 3D printing, injection molding, thermoforming, compression molding, turning, drilling, milling, and any combination thereof.

7. The liquid test sample dispensing device of claim 1 or 2, wherein each micro-cavity (46)/(346) of the plurality of micro-cavities (46)/(346) comprises at least one substantially three dimensional geometrical shape.

8. The liquid test sample dispensing device of claim 7, wherein the at least one substantially three dimensional geometrical shape is selected from the group consisting of a sphere, cylinder, cone, triangular prism, tetrahedron, square pyramid, cube, rectangular prism, pentagonal prism, hexagonal prism, heptagonal prism, octagonal prism, nonagonal prism, hendecagonal prism, dodecahedral prism, and any combinations thereof.

9. A method of collecting liquid waste produced as a by-product of at least one diagnostic assay, the method comprising the steps of:
collecting a patient's liquid test sample via a liquid test sample dispensing device, the liquid test sample dispensing device comprising:
a body (14)/ (314), the body (14)/ (314) comprising at least one side, a first end (38)/(354), a second end (42)/(362), and a plurality of micro-cavities (46)/(346), wherein the plurality of micro-cavities (46)/(346) is formed in the at least one side of the body (14)/(314), further wherein each micro-cavity (46)/(346) of the plurality of micro-cavities (46)/(346) comprise an opening (50)/(350) located on an outer portion of the at least one side of the body (14)/(314), the plurality of micro-cavities (46)/(346) being substantially located between the first end (38)/(354) and the second end (42)/(362) of the body (14)/(314); and
a capillary (18)/(318) that collects a patient's liquid test sample and dispenses the patient's liquid test sample, wherein a portion of the capillary (18)/(318) is disposed through the second end (42)/(362) of the body (14)/(314);
inserting the liquid test sample dispensing device into a reaction vessel;
dispensing the patient's liquid test sample from the capillary (18)/(318) of the liquid test sample dispensing device into the reaction vessel;
conducting at least one diagnostic assay; and
collecting a volume of liquid waste produced as a by-product of the at least one diagnostic assay, wherein the liquid waste is collected within the plurality of micro-cavities (46)/(346) by way of capillary action.

10. The method of claim 9, wherein the patient's liquid test sample is selected from the group consisting of whole blood and urine,

11. The method of claim 9, wherein the diagnostic assay is selected from the group consisting of a glycated hemoglobin diagnostic assay and a creatinine diagnostic assay.

12. The method of claim 9, wherein each micro-cavity of the plurality of micro-cavities (46)/(346) comprises at least one substantially three dimensional geometric shape.

13. The method of claim 12, wherein each micro-cavity of the plurality of micro-cavities (46)/(346) has a substantially geometric shape selected from the group consisting of a sphere, cylinder, cone, triangular prism, tetrahedron, square pyramid, cube, rectangular prism, pentagonal prism, hexagonal prism, heptagonal prism, octagonal prism, nonagonal prism, hendecagonal prism, dodecahedral prism, and any combinations thereof.

14. The method of claim 9, wherein the volume of liquid waste is about 600 microliters.

## Patentansprüche

1. Vorrichtung zur Ausgabe einer flüssigen Probe, Folgendes umfassend:
einen Körper (14), der Körper (14) mindestens eine Seite, ein erstes Ende (38), ein zweites Ende (42) und mehrere Mikrokavitäten (46) umfassend, wobei die mehreren Mikrokavitäten (46) in der mindestens einen Seite des Körpers (14) ausgebildet sind, wobei weiterhin jede Mikrokavität (46) der mehreren Mikrokavitäten (46) eine Öffnung (50) umfasst, die auf einem äußeren Abschnitt der mindestens einen Seite des Körpers (14) angeordnet ist, wobei die mehreren Mikrokavitäten (46) im Wesentlichen zwischen dem ersten Ende (38) und dem zweiten Ende (42) des Körpers (14) angeordnet sind; und
eine Kapillare (18) zum Sammeln einer flüssigen Probe eines Patienten und Ausgeben der flüssigen Probe des Patienten in einen Reaktionsbehälter für die Leitfähigkeit von mindestens einem Diagnose-Assay, wobei ein Abschnitt der Kapillare (18) durch das zweite Ende (42) des Körpers (14) angeordnet ist.

2. Vorrichtung zur Ausgabe einer flüssigen Probe, Folgendes umfassend:
einen Kolben (316), der Kolben einen Kolbenkörper (374) umfassend;
einen Körper (314), der Körper (314) mindestens eine Seite und eine Kolbenaufnahme (354) umfassend, die dazu ausgelegt ist, den Kolbenkörper (374) aufzunehmen, wobei eine Aufnahmewand mindestens eine Seite, ein erstes Ende (354), ein zweites Ende (362) und eine Bohrung (370) aufweist, die sich longitudinal von dem ersten Ende (354) zu dem zweiten Ende (362) der Aufnahmewand erstreckt, der Körper (314) weiterhin mehrere Mikrokavitäten (346) umfassend, wobei jede Mikrokavität (346) der mehreren Mikrokavitäten (346) eine Öffnung (350) umfasst, die auf einem äußeren Abschnitt der mindestens einen Seite des Körpers (314) angeordnet ist, wobei die mehreren Mikrokavitäten (346) weiterhin in der mindestens einen Seite des Körpers (314) derartig ausgebildet sind, dass sich die mehreren Mikrokavitäten (346) von der mindestens einen Seite des Körpers (314) einwärts in den Körper (314) erstrecken und die mehreren Mikrokavitäten (346) im Wesentlichen zwischen der mindestens einen Seite des Körpers (314) und der mindestens einen Seite der Aufnahmewand angeordnet sind; und
eine Kapillare (318) zum Sammeln einer flüssigen Probe eines Patienten und zum Ausgeben der flüssigen Probe des Patienten in einen Reaktionsbehälter für die Leitfähigkeit von mindestens einem Diagnose-Assay, wobei ein Abschnitt der Kapillare (318) durch das zweite Ende (362) des Körpers (314) und extern davon angeordnet ist.

3. Vorrichtung zur Ausgabe einer flüssigen Probe nach Anspruch 1 oder 2, wobei die flüssige Probe des Patienten aus der Gruppe ausgewählt ist, bestehend aus Vollblut und Urin.

4. Vorrichtung zur Ausgabe einer flüssigen Probe nach Anspruch 1 oder 2, wobei das Diagnose-Assay aus der Gruppe ausgewählt ist, bestehend aus einem Diagnose-Assay für glykosyliertes Hämoglobin und einem Diagnose-Assay für Kreatinin.

5. Vorrichtung zur Ausgabe einer flüssigen Probe nach Anspruch 1 oder 2, wobei der Körper (14)/(314) und die Kapillare (18)/(318) aus einem Material aufgebaut sind, das aus der Gruppe ausgewählt ist, bestehend aus synthetischen, natürlich vorkommenden oder daraus abgeleiteten Polymeren, organischen Polymeren, anorganischen Polymeren, thermoplastischem Polymer(en), duroplastischem Polymer(en), Elastomer(en), synthetischer Faser(n), Polyethylen niedriger Dichte, Polyethylen hoher Dichte, Polystyrol, Polyvinylchlorid, Styrol-Butadien, Acryl(en), Polyacrylen, Polyvinylacetat und Kombinationen davon.

6. Vorrichtung zur Ausgabe einer flüssigen Probe nach Anspruch 1 oder 2, wobei die Mikrokavitäten (46)/(346) in der mindestens einen Seite des Körpers (14)/(314) über Fertigungstechniken ausgebildet sind, die aus der Gruppe ausgewählt sind, bestehend aus 3D-Drucken, Spritzgießen, Warmformen, Pressformen, Drehen, Bohren, Fräsen und jede Kombination davon.

7. Vorrichtung zur Ausgabe einer flüssigen Probe nach Anspruch 1 oder 2, wobei jede Mikrokavität (46)/(346) der mehreren Mikrokavitäten (46)/(346) mindestens eine im Wesentlichen dreidimensionale geometrische Gestalt umfasst.

8. Vorrichtung zur Ausgabe einer flüssigen Probe nach Anspruch 7, wobei die mindestens eine im Wesentlichen dreidimensionale geometrische Gestalt aus der Gruppe ausgewählt ist, bestehend aus einer Kugel, einem Zylinder, einem Kegel, einem dreieckigen Prisma, einem Tetraeder, einer quadratischen Pyramide, einem Würfel, einem rechteckigen Prisma, einem pentagonalen Prisma, einem hexagonalen Prisma, einem heptagonalen Prisma, einem oktogonalen Prisma, einem nonagonalen Prisma, einem undekanalen Prisma, einem dodekaedrischen Prisma und jeder Kombinationen davon.

9. Verfahren zum Sammeln von flüssigem Abfall, der als ein Nebenprodukt von mindestens einem Diagnose-Assay hergestellt wird, das Verfahren die folgenden Schritte umfassend:
Sammeln einer flüssigen Probe eines Patienten über eine Vorrichtung zur Ausgabe einer flüssigen Probe, die Vorrichtung zur Ausgabe einer flüssigen Probe Folgendes umfassend:
einen Körper (14)/(314), der Körper (14)/(314) mindestens eine Seite, ein erstes Ende (38)/(354), ein zweites Ende (42)/(362) und mehrere Mikrokavitäten (46)/(346) umfassend, wobei die mehreren Mikrokavitäten (46)/(346) in der mindestens einen Seite des Körpers (14)/(314) ausgebildet sind, wobei weiterhin jede Mikrokavität (46)/(346) der mehreren Mikrokavitäten (46)/(346) eine Öffnung (50)/(350) umfasst, die auf einem äußeren Abschnitt der mindestens einen Seite des Körpers (14)/(314) angeordnet ist, wobei die mehreren Mikrokavitäten (46)/(346) im Wesentlichen zwischen dem ersten Ende (38)/(354) und dem zweiten Ende (42)/(362) des Körpers (14)/(314) angeordnet sind; und
eine Kapillare (18)/(318), die eine flüssige Probe eines Patienten sammelt und die flüssige Probe des Patienten ausgibt, wobei ein Abschnitt der Kapillare (18)/(318) durch das zweite Ende (42)/(362) des Körpers (14)/(314) angeordnet ist;
Einführen der Vorrichtung zur Ausgabe einer flüssigen Probe in einen Reaktionsbehälter;
Ausgeben der flüssigen Probe des Patienten aus der Kapillare (18)/(318) der Vorrichtung zur Ausgabe einer flüssigen Probe in den Reaktionsbehälter;
Ausführen von mindestens einem Diagnose-Assay; und
Sammeln eines Volumens von flüssigem Abfall, der als ein Nebenprodukt des mindestens einen Diagnose-Assays hergestellt wurde, wobei der flüssige Abfall in den mehreren Mikrokavitäten (46)/(346) mittels Kapillarwirkung gesammelt wird.

10. Verfahren nach Anspruch 9, wobei die flüssige Probe des Patienten aus der Gruppe ausgewählt ist, bestehend aus Vollblut und Urin.

11. Verfahren Anspruch 9, wobei das Diagnose-Assay aus der Gruppe ausgewählt ist, bestehend aus einem Diagnose-Assay für glykosyliertes Hämoglobin und einem Diagnose-Assay für Kreatinin.

12. Verfahren nach Anspruch 9, wobei jede Mikrokavität der mehreren Mikrokavitäten (46)/(346) mindestens eine im Wesentlichen dreidimensionale geometrische Gestalt umfasst.

13. Verfahren nach Anspruch 12, wobei jede Mikrokavität der mehreren Mikrokavitäten (46)/(346) eine im Wesentlichen geometrische Form aufweist, die aus der Gruppe ausgewählt ist, bestehend aus einer Kugel, einem Zylinder, einem Kegel, einem dreieckigen Prisma, einem Tetraeder, einer quadratischen Pyramide, einem Würfel, einem rechteckigen Prisma, einem pentagonalen Prisma, einem hexagonalen Prisma, einem heptagonalen Prisma, einem oktogonalen Prisma, einem nonagonalen Prisma, einem undekanalen Prisma, einem dodekaedrischen Prisma und jeder Kombinationen davon.

14. Verfahren nach Anspruch 9, wobei das Volumen des flüssigen Abfalls ungefähr 600 Mikroliter ist.

## Revendications

1. Dispositif de distribution d'échantillon test liquide, comprenant :
un corps (14), le corps (14) comprenant au moins un côté, une première extrémité (38), une seconde extrémité (42) et une pluralité de microcavités (46), dans lequel la pluralité de micro-cavités (46) est formée dans le au moins un côté du corps (14), dans lequel en outre chaque micro-cavité (46) de la pluralité de micro-cavités (46) comprend une ouverture (50) située sur une partie extérieure du au moins un côté du corps (14), la pluralité de micro-cavités (46) étant sensiblement située entre la première extrémité (38) et la seconde extrémité (42) du corps (14) ; et
un capillaire (18) pour collecter un échantillon test liquide d'un patient et distribuer l'échantillon test liquide d'un patient dans un récipient de réaction pour la réalisation d'au moins un essai de diagnostic, dans lequel une partie du capillaire (18) est disposée à travers la seconde extrémité (42) du corps (14).

2. Dispositif de distribution d'échantillon test liquide, comprenant :
un piston (316), le piston comprenant un corps de piston (374) ;
un corps (314), le corps (314) comprenant au moins un côté et un réceptacle de piston (354) configuré pour recevoir le corps de piston (374), une paroi de réceptacle présentant au moins un côté, une première extrémité (354), un seconde extrémité (362), et un alésage (370) s'étendant longitudinalement de la première extrémité (354) à la seconde extrémité (362) de la paroi de réceptacle, le corps (314) comprenant en outre une pluralité de micro-cavités (346), dans lequel chaque micro-cavité (346) de la pluralité de micro-cavités (346) comprend une ouverture (350) située sur une partie extérieure du au moins un côté du corps (314), dans lequel en outre la pluralité de micro-cavités (346) sont formées dans le au moins un côté du corps (314) de telle sorte que la pluralité de microcavités (346) s'étendent vers l'intérieur dans le corps (314) depuis le au moins un côté du corps (314) et la pluralité de micro-cavités (346) sont sensiblement situées entre le au moins un côté du corps (314) et le au moins un côté de la paroi de réceptacle ; et
un capillaire (318) pour collecter un échantillon test liquide d'un patient et distribuer l'échantillon test liquide du patient dans un récipient de réaction pour la réalisation d'au moins un essai de diagnostic, dans lequel une partie du capillaire (318) est disposée à travers la seconde extrémité (362) du corps (314), et à l'extérieur de celle-ci.

3. Dispositif de distribution d'échantillon test liquide selon la revendication 1 ou 2, dans lequel l'échantillon test liquide du patient est choisi dans le groupe consistant en du sang total et de l'urine.

4. Dispositif de distribution d'échantillon test liquide selon la revendication 1 ou 2, dans lequel l'essai de diagnostic est choisi dans le groupe consistant en un essai de diagnostic d'hémoglobine glyquée et un essai de diagnostic de créatinine.

5. Dispositif de distribution d'échantillon test liquide selon la revendication 1 ou 2, dans lequel le corps (14)/(314) et le capillaire (18)/(318) sont constitués d'un matériau choisi dans le groupe consistant en polymères synthétiques, naturels ou dérivés, polymères organiques, polymères inorganiques, polymère(s) thermoplastique(s), polymère(s) thermodurcissable(s), élastomère(s), fibre(s) synthétique(s), polyéthylène basse densité, polyéthylène haute densité, polystyrène, chlorure de polyvinyle, styrène butadiène, acrylique(s), polyacryliques, acétate de polyvinyle, et des combinaisons de ceux-ci.

6. Dispositif de distribution d'échantillon test liquide selon la revendication 1 ou 2, dans lequel les micro-cavités (46)/(346) sont formées dans le au moins un côté du corps (14)/(314) par l'intermédiaire de techniques de fabrication choisies dans le groupe consistant en impression 3D, moulage par injection, thermoformage, moulage par compression, tournage, perçage, fraisage et toute combinaison de ceux-ci.

7. Dispositif de distribution d'échantillon test liquide selon la revendication 1 ou 2, dans lequel chaque micro-cavité (46)/(346) de la pluralité de micro-cavités (46)/(346) comprend au moins une forme géométrique sensiblement tridimensionnelle.

8. Dispositif de distribution d'échantillon test liquide selon la revendication 7, dans lequel la au moins une forme géométrique sensiblement tridimensionnelle est choisie dans le groupe consistant en une sphère, un cylindre, un cône, un prisme triangulaire, un tétraèdre, une pyramide carrée, un cube, un prisme rectangulaire, un prisme pentagonal, un prisme hexagonal, un prisme heptagonal, un prisme octogonal, un prisme nonagonal, un prisme hendécagonal, un prisme dodécaédrique, et toutes combinaisons de ceux-ci.

9. Procédé de collecte de déchets liquides produits en tant que sous-produit d'au moins un essai de diagnostic, le procédé comprenant les étapes consistant à :
collecter un échantillon test liquide d'un patient via un dispositif de distribution d'échantillon test liquide, le dispositif de distribution d'échantillon test liquide comprenant :
un corps (14)/(314), le corps (14)/(314)comprenant au moins un côté, une première extrémité (38)/(354), une seconde extrémité (42)/(362), et une pluralité de micro-cavités (46)/(346), dans lequel la pluralité de microcavités (46)/(346) est formée dans le au moins un côté du corps (14)/(314), dans lequel en outre chaque micro-cavité (46)/(346) de la pluralité de microcavités (46)/(346) comprend une ouverture (50)/(350) située sur une partie extérieure du au moins un côté du corps (14)/(314), la pluralité de microcavités (46)/(346) étant sensiblement située entre la première extrémité (38)/(354) et la seconde extrémité (42)/(362) du corps (14)/(314) ; et
un capillaire (18)/(318) qui collecte un échantillon test liquide d'un patient et distribue l'échantillon test liquide d'un patient, dans lequel une partie du capillaire (18)/(318) est disposée à travers la seconde extrémité (42)/(362) du corps (14)/(314) ;
insérer le dispositif de distribution d'échantillon test liquide dans un récipient de réaction ;
distribuer l'échantillon test liquide d'un patient depuis le capillaire (18)/(318) du dispositif de distribution d'échantillon test liquide dans le récipient de réaction ;
réaliser au moins un essai de diagnostic ; et
collecter un volume de déchets liquides produits en tant que sous-produit du au moins un essai de diagnostic, dans lequel les déchets liquides sont collectés dans la pluralité de microcavités (46)/(346) par action capillaire.

10. Procédé selon la revendication 9, dans lequel l'échantillon test liquide du patient est choisi dans le groupe consistant en du sang total et de l'urine.

11. Procédé selon la revendication 9, dans lequel l'essai de diagnostic est choisi dans le groupe consistant en un essai de diagnostic d'hémoglobine glyquée et un essai de diagnostic de créatinine.

12. Procédé selon la revendication 9, dans lequel chaque micro-cavité de la pluralité de micro-cavités (46)/(346) comprend au moins une forme géométrique sensiblement tridimensionnelle.

13. Procédé selon la revendication 12, dans lequel chaque micro-cavité de la pluralité de micro-cavités (46)/(346) présente une forme sensiblement géométrique choisie dans le groupe consistant en une sphère, un cylindre, un cône, un prisme triangulaire, un tétraèdre, une pyramide carrée, un cube, un prisme rectangulaire, un prisme pentagonal, un prisme hexagonal, un prisme heptagonal, un prisme octogonal, un prisme nonagonal, un prisme hendécagonal, un prisme dodécaédrique, et toutes combinaisons de ceux-ci.

14. Procédé selon la revendication 9, dans lequel le volume de déchets liquides est d'environ 600 microlitres.
